# EUROPEAN PATENT SPECIFICATION

(11) **EP 4 310 085 B1**
(45) Date of publication and mention of the grant of the patent: **02.12.2026**
(21) Application number: 22771769.1
(22) Date of filing: 16.03.2022
(51) Int. Cl.: C07D 471/04, C07C 55/06, C07C 309/04, C07C 309/19, C07C 309/35, A61K 31/437, A61P 29/00, A61P 17/00, A61P 1/00, A61P 1/04

(54) **N-(4-(1-(2-CYANOACETYL)-3-METHYL-1,2,3,6-TETRAHYDROPYRIDIN-4-YL)-1H-PYRROLO[2,3-B]PYRIDIN-6-YL)CYCLOPROPANECARBOXAMIDE SALTS AS JAK INHIBITORS FOR THE TREATMENT OF ATOPIC DERMATITIS OR INFLAMMATORY BOWEL DISEASE**
N-(4-(1-(2-CYANOACETYL)-3-METHYL-1,2,3,6-TETRAHYDROPYRIDIN-4-YL)-1H-PYRROLO[2,3-B]PYRIDIN-6-YL)CYCLOPROPANECARBOXAMID-SALZE ALS JAK-INHIBITOREN ZUR BEHANDLUNG VON ATOPISCHER DERMATITIS ODER ENTZÜNDLICHEN DARMERKRANKUNGEN
SELS DE N-(4-(1-(2-CYANOACÉTYL)-3-MÉTHYL-1,2,3,6-TÉTRAHYDROPYRIDIN-4-YL)-1H-PYRROLO[2,3-B]PYRIDIN-6-YL)CYCLOPROPANECARBOXAMIDE EN TANT QU'INHIBITEURS DE JAK POUR LE TRAITEMENT DE LA DERMATITE ATOPIQUE OU DES MALADIES INFLAMMATOIRES DE L'INTESTIN

(30) Priority: 16.03.2021 KR 20210034296; 14.04.2021 KR 20210048814
(43) Date of publication of application: 24.01.2024
(60) Divisional application: 26212074.4; 26212076.9
(73) Proprietor: HK inno.N Corporation, Cheongju-si, Chungcheongbuk-do 28158 (KR)
(72) Inventor: LEE, Juhyun, Seoul 04551 (KR); KIM, Young Ju, Seoul 04551 (KR); KIM, Jun, Seoul 04551 (KR); KIM, Hanna, Seoul 04551 (KR); CHOI, Jong Ryoul, Seoul 04551 (KR); KWEON, Jae Hong, Seoul 04551 (KR); KIM, Dongkyu, Seoul 04551 (KR); BYEON, Yeji, Seoul 04551 (KR); SHIN, Hyunwoo, Seoul 04551 (KR); YOON, Daseul, Seoul 04551 (KR); LEE, Ye-Lim, Seoul 04551 (KR); JUNG, Seung Hee, Seoul 04551 (KR); HWANG, Do Seok, Seoul 04551 (KR)
(74) Representative: HGF
(86) International application number: PCT/KR2022/003702
(87) International publication number: WO 2022/197103

(56) References cited:
- WO-A1-2011/003418
- WO-A1-2017/087905
- KR-A- 20140 004 637
- KR-A- 20140 027 318
- KR-A- 20190 043 437

## Description

### Technical Field

The present invention relates to a novel salt of a heterocyclic compound as a protein kinase inhibitor and a use thereof.

References in the present description to methods of medical treatment or medical uses have to be understood as references to the compounds of the present invention for use in methods of medical treatment. Methods of medical treatment or medical uses are not encompassed by the claimed invention.

### Background

In general, it is obvious that the same drug may show a difference in pharmaceutically important properties such as solubility, dissolution properties, and bioavailability in each form of amorphous, one or more crystalline forms, salts, and the like. The types of pharmaceutically acceptable salts of drugs are well known and studied a lot, but even the same salt has different properties for each drug, and it is difficult to obtain an ideal salt satisfying all the properties which should be considered pharmaceutically important, such as solubility and stability.

Janus kinase (JAK) is an enzyme which controls various intracellular processes by phosphorylating other proteins to regulate the activity, position, and function of the proteins. The Janus kinase is located at an intracellular receptor of an inflammatory cytokine, and the inflammatory cytokine binds with the receptor, phosphorylates, and transmits a signal of the inflammatory cytokine into cells through an action with STAT molecules. An excessive activation of signal transduction through such various inflammatory cytokines causes an immune system of our body to attack the body and thus leads to the occurrence of autoimmune diseases. In recent years, it has been reported that JAK1 inhibitors rapidly ameliorate the severity and symptoms of Alzheimer's disease in phase II and phase III clinical trials on selective JAK1 inhibitors, upadasitinib and abrocitinib.

Meanwhile, atopic dermatitis (AD) is one of the most common chronic inflammatory skin diseases. Elevated levels of Th(T helper)2, Th22 and some Th1 and Th17 cytokines induce abnormal immune activation in skin lesions of atopic dermatitis. Recently, the use of immune-targeted therapeutics has been increasing for the treatment of atopic dermatitis, and dupilumab, a monoclonal antibody targeting the IL-4 receptor, has recently been approved as a therapeutic agent for atopic dermatitis. As the number of patients with atopic dermatitis continues to increase, there has been a continuous demand for therapeutic agents having excellent therapeutic efficacy.

### Detailed Description of the Invention

The present description refers to the disclosure and not necessarily to the claimed invention. References to "the present invention" must be understood as references to the disclosure, and not necessarily to the claimed invention. The scope of the claimed invention is defined by the claims.

### Technical Problem

One object of the present invention is to provide a novel salt of a heterocyclic compound as a protein kinase inhibitor.

Another object of the present invention is to provide a pharmaceutical composition for treating or preventing atopic dermatitis, which includes a heterocyclic compound or pharmaceutically acceptable salts thereof as a protein kinase inhibitor.

Still another object of the present invention is to provide a pharmaceutical composition for treating or preventing inflammatory bowel disease, which includes a heterocyclic compound or pharmaceutically acceptable salts thereof as a protein kinase inhibitor.

Still another object of the present invention is to provide a use of a heterocyclic compound or pharmaceutically acceptable salts thereof as a protein kinase inhibitor for treating or preventing atopic dermatitis.

Still another object of the present invention is to provide a use of a heterocyclic compound or pharmaceutically acceptable salts thereof as a protein kinase inhibitor for treating or preventing inflammatory bowel disease.

Still another object of the present invention is to provide a use of a heterocyclic compound or pharmaceutically acceptable salts thereof as a protein kinase inhibitor for preparing a medicament for treating or preventing atopic dermatitis.

Still another object of the present invention is to provide a use of a heterocyclic compound or pharmaceutically acceptable salts thereof as a protein kinase inhibitor for preparing a medicament for treating or preventing inflammatory bowel disease.

Still another object of the present invention is to provide a method for treating or preventing atopic dermatitis, which includes administering a heterocyclic compound or pharmaceutically acceptable salts thereof as a protein kinase inhibitor into an individual.

Still another object of the present invention is to provide a method for treating or preventing inflammatory bowel disease, which includes administering a heterocyclic compound or pharmaceutically acceptable salts thereof as a protein kinase inhibitor into an individual.

### Technical Solution

The inventors of the present application have made efforts to discover a compound having improved physicochemical properties, capable of minimizing the occurrence of related materials by enhancing stability against heat and moisture, while having pharmacological activity equal to or higher than that of an existing compound, and thus have confirmed that the salts of the heterocyclic compound according to the present invention have low hygroscopicity and excellent physicochemical properties, and thus are stable against heat and moisture along with an improved effect of solubility, thereby completing the present invention.

In addition, the inventors have discovered a use of the heterocyclic compound and pharmaceutically acceptable salts thereof according to the present invention for treating atopic dermatitis and inflammatory bowel disease.

The heterocyclic compound and pharmaceutically acceptable salts thereof according to the present invention were able to more effectively inhibit the atopic dermatitis-like skin severity by oral and topical administration in a mouse model with atopic dermatitis than other selective JAK1 inhibitors. In addition, in vitro human whole blood assays have demonstrated that the heterocyclic compound of the invention is the best selective JAK1 inhibition for advantageous safety compared to other JAK inhibitors. Furthermore, it could be confirmed that the heterocyclic compound and pharmaceutically acceptable salts thereof according to the present invention have a therapeutic effect even in an inflammatory bowel disease model.

### Novel salt and method for preparing same

In the novel salt of the heterocyclic compound as a protein kinase inhibitor according to the present invention, the heterocyclic compound is represented by formula I below.

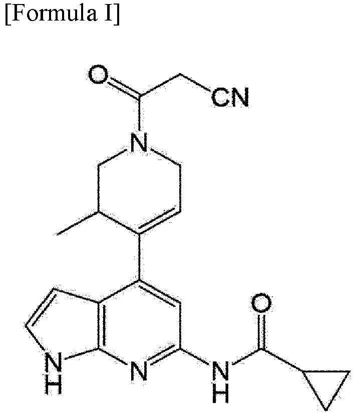

The name of the compound represented by above formula I is N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

The heterocyclic compound of above formula I may refer to each of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide and (R)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, or may be a mixture thereof.

In one embodiment, the heterocyclic compound or pharmaceutically acceptable salts thereof in the present invention may be a compound represented by formula II below or pharmaceutically acceptable salts thereof.

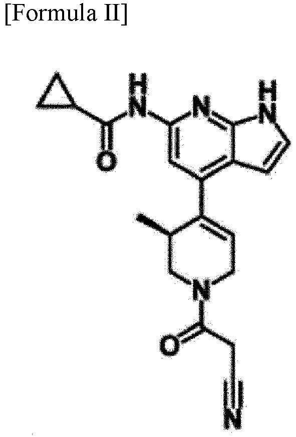

The name of the heterocyclic compound represented by above formula II is (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

In the present invention, the pharmaceutically acceptable salt of the heterocyclic compound represented by above formula I may be an organic acid salt of the heterocyclic compound represented by above formula I. In this case, the organic acid salt includes hydrochloride salt, hydrobromide salt, mesylate salt, phosphate salt, napadisylate salt, camsylate salt, or oxalate salt.

In the present invention, the pharmaceutically acceptable salt of the heterocyclic compound represented by above formula I may be an organic acid salt including N-(4-(1-(2-cyanoacetyl)-3-methyl-1,2,3,6-tetrahydropyridin-4-yl)-1H-pyrrolo[2,3-b]pyridin-6-yl)cyclopropanecarboxamide and organic acid, in which the organic acid salt may be hydrochloride salt, hydrobromide salt, mesylate salt, phosphate salt, napadisylate salt, camsylate salt, or oxalate salt.

The organic acid salt of the present invention may include N-(4-(1-(2-cyanoacetyl)-3-methyl-1,2,3,6-tetrahydropyridin-4-yl)-1H-pyrrolo[2,3-b]pyridin-6-yl)cyclopropanecarboxamide and organic salt at an equivalence ratio of 1:1 to 1:1.3.

In the present invention, the pharmaceutically acceptable salts of the heterocyclic compound represented by above formula I may be obtained with high purity.

In addition, in the present invention, the pharmaceutically acceptable salts of the heterocyclic compound represented by above formula I may exhibit excellent stability even under the conditions of high temperature and high humidity, also have excellent photostability, and thus may be stably maintained against heat and moisture for a long period of time. Furthermore, in the present invention, the pharmaceutically acceptable salts of the heterocyclic compound represented by above formula I may exhibit excellent solubility and physicochemical properties, and thus have excellent bioavailability. Moreover, in the present invention, the pharmaceutically acceptable salts of the heterocyclic compound represented by above formula I may have low hygroscopicity and may not absorb surrounding moisture, and thus may be maintained without a change in moisture content for a long period of time.

The salt of the heterocyclic compound represented by formula I of the present invention may be administered in an oral or transdermal form.

The salt of the heterocyclic compound represented by formula I of the present invention may treat or prevent atopic dermatitis.

The salt of the heterocyclic compound represented by formula I of the present invention may treat or prevent inflammatory bowel disease. The inflammatory bowel disease may include ulcerative colitis or Crohn's disease.

In the present invention, a method for preparing the pharmaceutically acceptable salts of the heterocyclic compound represented by above formula I or II may include:
(A) dissolving or suspending the heterocyclic compound represented by above formula I in an organic solvent;
(B) adding and stirring any one organic acid selected from hydrochloric acid, bromic acid, methanesulfonic acid, phosphoric acid, 1,5-naphthalenedisulfonic acid, camphorsulfonic acid, and oxalic acid to form a salt of the heterocyclic compound; and
(C) solidifying the salt formed in the previous step by stirring.

The preparation method may further include (D) adding an anti-solvent to mature the solid.

In above (A), the heterocyclic compound represented by above formula I or II may be an amorphous or crystalline form.

The organic solvent of above (A) may include ethyl acetate, acetone, methanol, ethanol, isopropanol, acetonitrile, 2-butanone, or a mixture thereof.

In above (B), N-(4-(1-(2-cyanoacetyl)-3-methyl-1,2,3,6-tetrahydropyridin-4-yl)-1H-pyrrolo[2,3-b]pyridin-6-yl)cyclopropanecarboxamide and organic salt may be mixed at an equivalence ratio of 1:1 to 1:1.3.

### Use for treating or preventing atopic dermatitis

**(1)** The present invention provides a composition for treating or preventing atopic dermatitis, which includes N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, which is a heterocyclic compound represented by formula I, or pharmaceutically acceptable salts thereof as an active ingredient.
**(2)** The present invention provides a method for treating or preventing atopic dermatitis, which includes administering a pharmaceutical composition including a heterocyclic compound represented by formula I or pharmaceutically acceptable salts thereof.
**(3)** The present invention provides a use of a heterocyclic compound represented by formula I or pharmaceutically acceptable salts thereof for treating or preventing atopic dermatitis.
**(4)** The present invention provides a use of a heterocyclic compound represented by formula I or pharmaceutically acceptable salts thereof for producing a medicament for preventing or treating atopic dermatitis.
**(5)** In the present invention according to above **(1), (2), (3)** or **(4),** "atopic dermatitis" is meant to include all diseases classified as atopic dermatitis in the art, regardless of whether they occur directly or indirectly. Usually, atopic dermatitis is classified into early childhood atopic dermatitis, pediatric atopic dermatitis, adult atopic dermatitis, and pregnant women's atopic dermatitis according to the onset time of the dermatitis or the subject of the invention. In the present invention, atopic dermatitis is defined to include all of these types of atopic dermatitis.
**(6)** In the present invention according to above **(1), (2), (3), (4)** or **(5),** the pharmaceutically acceptable salts may be hydrochloride salt, hydrobromide salt, phosphate salt, camsylate salt, oxalate salt, mesylate salt, or napadisylate salt.

### Use for treating or preventing inflammatory bowel disease

**(7)** The present invention provides a composition for treating or preventing inflammatory bowel disease, which includes N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, which is a heterocyclic compound represented by formula I, or pharmaceutically acceptable salts thereof as an active ingredient.

**(8)** The present invention provides a method for treating or preventing inflammatory bowel disease, which includes administering a pharmaceutical composition including the heterocyclic compound represented by formula I or pharmaceutically acceptable salts thereof.

**(9)** The present invention provides a use of the heterocyclic compound represented by formula I or pharmaceutically acceptable salts thereof for treating or preventing inflammatory bowel disease.

**(10)** The present invention provides a use of the heterocyclic compound represented by formula I or pharmaceutically acceptable salts thereof for producing a medicament for treating or preventing inflammatory bowel disease.

**(11)** In the present invention according to above **(7), (8), (9)** or **(10),** "inflammatory bowel disease" means chronic inflammation which occurs to the bowels without any known cause, and may usually refer to ulcerative colitis and Crohn's disease, which are idiopathic inflammatory bowel diseases, but may also include intestinal Behcet's disease which is relatively common in Korea. In addition, inflammatory bowel disease is defined as a collective term for inflammatory diseases which occur to all intestines, such as infectious enteritis including bacterial, viral, amoebic, tuberculous enteritis and the like, ischemic bowel disease, radiation enteritis, etc.

**(12)** In the present invention according to above **(7), (8), (9), (10)** or **(11),** the pharmaceutically acceptable salts may be hydrochloride salt, hydrobromide salt, phosphate salt, camsylate salt, oxalate salt, mesylate salt, or napadisylate salt.

The pharmaceutical composition for preventing or treating atopic dermatitis or inflammatory bowel disease of the present invention according to above **(1)** or **(7)** may further include pharmaceutically acceptable additives, conventionally used appropriate carriers, excipients, disintegrants, binders, glidants or diluents.

Said "pharmaceutically acceptable additives" may include carriers, excipients, disintegrants, binders, glidants or diluents, which neither irritate organisms nor inhibit the biological activity and properties of an injected compound. Said types of additives usable in the present invention are not particularly limited, and any additives may be used, as long as they are conventionally used in the art and pharmaceutically acceptable. A non-limiting example of said additives may include mannitol, microcrystalline cellulose, croscarmellose sodium, hydroxypropyl cellulose, colloidal silicon dioxide, magnesium stearate or mixtures thereof. In addition, such additives may be used with the addition of other conventional additives, such as antioxidants, buffer solutions, bacteriostatic agents and/or the like, if necessary.

In the present invention, the compound having an effect of treating or preventing atopic dermatitis or inflammatory bowel disease may be N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, which is the heterocyclic compound represented by formula I, or pharmaceutically acceptable salts thereof, in which the salt may be hydrochloride salt, hydrobromide salt, phosphate salt, camsylate salt, oxalate salt, mesylate salt, or napadisylate salt.

In the present invention, the heterocyclic compound represented by formula I or pharmaceutically acceptable salts thereof may be administered in an oral or transdermal form.

In the present invention, a dosage of the heterocyclic compound represented by formula I or pharmaceutically acceptable salts thereof may be a pharmaceutically effective amount. The "pharmaceutically effective amount" may mean an amount enough to prevent or treat diseases at a reasonable benefit/risk ratio applicable to medical treatment, and a level of effective dose may be variously selected by those skilled in the art according to factors such as a formulation method, a patient's condition, weight, gender and age, a degree of disease, a drug form, an administration route and period, an excretion rate, reaction sensitivity, etc. The effective amount may vary depending on a route of disposal, a use of excipient, and possibility of being used with other drugs, as recognized by those skilled in the art.

In one embodiment, the heterocyclic compound represented by formula I of the present invention or pharmaceutically acceptable salts thereof may be administered in a dose of 0.1 to 5% as a transdermal agent and 10 to 200 mg/kg as an oral agent. As one example, the heterocyclic compound represented by formula I of the present invention or pharmaceutically acceptable salts thereof may be administered in a dose of 0.3 to 3% as a transdermal agent and 50 to 200 mg/kg as an oral agent.

As one example, the heterocyclic compound represented by formula I of the present invention or pharmaceutically acceptable salts thereof may be orally administered in a dose of 10 to 180 mg/kg.

If formulated into a preparation for oral administration, the heterocyclic compound represented by formula I of the present invention or pharmaceutically acceptable salts thereof may be formulated into a preparation, such as tablets, troches, lozenges, water-soluble or oil suspensions, prepared powders or granules, emulsions, hard or soft capsules, syrup, elixirs or the like.

If formulated into a transdermal agent, the heterocyclic compound represented by formula I of the present invention or pharmaceutically acceptable salts thereof may be formulated into a dosage form, for example, solution, suspension, gel, cream, emulsion, etc.

### Advantageous Effects

A novel salt of the heterocyclic compound represented by formula I according to the present invention has excellent water solubility and excellent physical and chemical stability and thus can be usefully employed in formulating medicinal products.

In addition, the present invention uses the heterocyclic compound represented by formula I or pharmaceutically acceptable salts thereof to effectively inhibit Janus kinase, which is the receptor of inflammatory cytokines, and thus can effectively treat and prevent atopic dermatitis and inflammatory bowel disease.

### Brief Description of the Drawings

FIG. 1 is a graph showing the results of NMR analysis on hydrochloride salt of the compound represented by formula II of the present invention.
FIG. 2 is a graph showing the results of NMR analysis on hydrobromide salt of the present invention.
FIG. 3 is a graph showing the results of NMR analysis on phosphate salt of the present invention.
FIG. 4 is a graph showing the results of NMR analysis on camsylate salt of the present invention.
FIG. 5 is a graph showing the results of NMR analysis on oxalate salt of the present invention.
FIGS. 6 and 7 are graphs showing the results of NMR analysis on mesylate salt, oxalate salt and napadisylate salt of the present invention.
FIG. 8 is a graph showing the results of an experiment on IL-4 secretion inhibitory activity in Basophil cells with regard to Compound 1.
FIG. 9 is a view showing graphs of the evaluation results of IL-13, IL-10, and TNF-α secretion activity with regard to Compound 1 of the present invention.
FIG. 10 is a view for explaining a DNCB-induced animal model in Experimental Example 6.
FIGS. 11 to 16 are views showing the results of identifying a therapeutic effect on atopic dermatitis in a DNCB-induced animal model in Experimental Example 6.
FIGS. 17 to 19 are views showing the results of identifying a therapeutic effect on atopic dermatitis in a DNCB-induced animal model in Experimental Example 7.
FIG. 20 is a view for explaining a DSS-induced animal model in Experimental Example 8.
FIGS. 21 to 24 are views showing the results of identifying a therapeutic effect on colitis in a DSS-induced animal model in Experimental Example 8.
FIG. 25 is a view for explaining a DNBS-induced animal model in Experimental Example 9.
FIG. 26 is a view showing the results of identifying a therapeutic effect on colitis in a DNBS-induced animal model in Experimental Example 9.
FIG. 27 is a view for explaining a HDM-induced animal model in Experimental Example 10.
FIGS. 28 to 30 are views showing the results of identifying a therapeutic effect on atopic dermatitis in a HDM-induced animal model in Experimental Example 10.

### Best Mode for Invention

Hereinafter, the present invention will be described with reference to examples. All the terms used herein including technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

In each of the drawings, *p<0.05, **p<0.01, ***p<0.001.

### Preparation Example 1: Synthesis of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide

The title compound was prepared according to the method disclosed in Korean Unexamined Patent Application No. 2019-0043437.

¹H NMR (400 MHz, DMSO-d₆) δ 11.44 (s, 1H), 10.57 (s, 1H), 7.84 (d, J = 10.2 Hz, 1H), 7.34 (d, J = 3.1 Hz, 1H), 6.48 (dd, J = 1.8, 3.7 Hz, 1H), 6.17 - 6.03 (m, 1H), 4.31 - 4.01 (m, 6H), 3.96 - 3.62 (m, 2H), 3.02 (m J = 36.6 Hz, 1H), 2.02 (s, 1H), 0.88 (s, 3H), 0.84 - 0.73 (m, 4H); MS(ESI+) m/z 364 (M+H)⁺

### Example 1: Preparation of hydrochloride salt

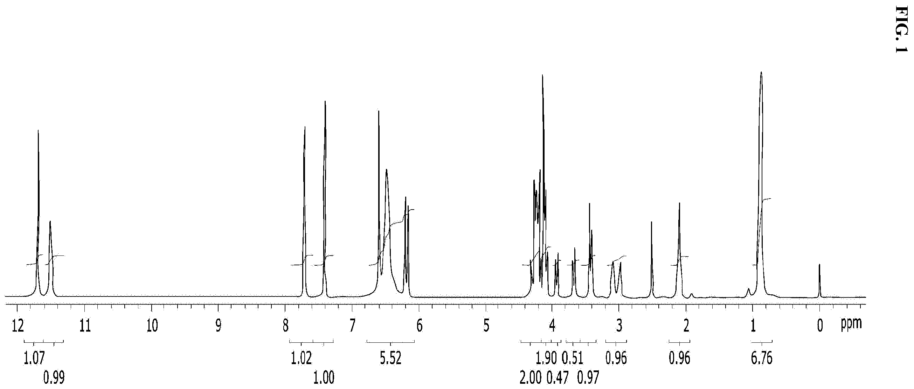

90 mL of methanol was added to 30 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, stirred and heated up to 50°C. Then, 1.1 eq. of hydrochloric acid (in MeOH) was slowly added dropwise thereto and stirred at 50°C for two hours to obtain a solution. The solution was cooled to room temperature and precipitated. Then, it was then filtered, washed with methanol, and vacuum-dried to obtain the title compound (amount obtained 31.1 g, yield 94%) as a yellow powder. NMR analysis was performed to confirm the formation of the title compound. The results thereof were shown in FIG. 1.

### Example 2: Preparation of hydrobromide salt

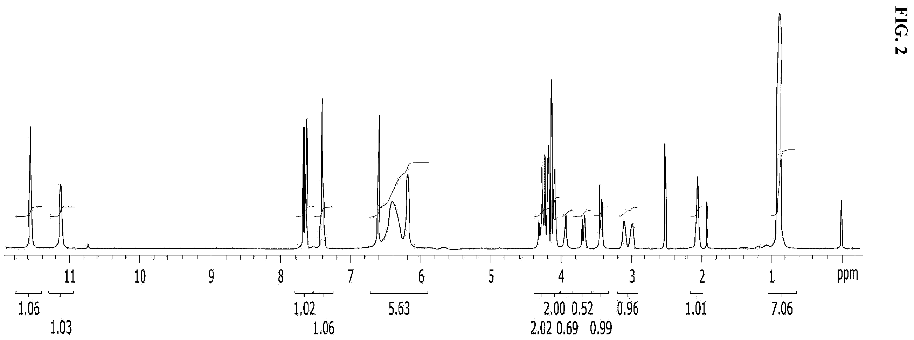

300 mL of acetone was added to 30 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, and stirred. Then, 1.1 eq. of hydrobromic acid was slowly added dropwise thereto, stirred, filtered, washed with methanol, and vacuum-dried to obtain the title compound (amount obtained 34.4 g, yield 94%) as a yellow powder. NMR analysis was performed to confirm the formation of the title compound. The results thereof were shown in FIG. 2.

### Example 3: Preparation of phosphate salt

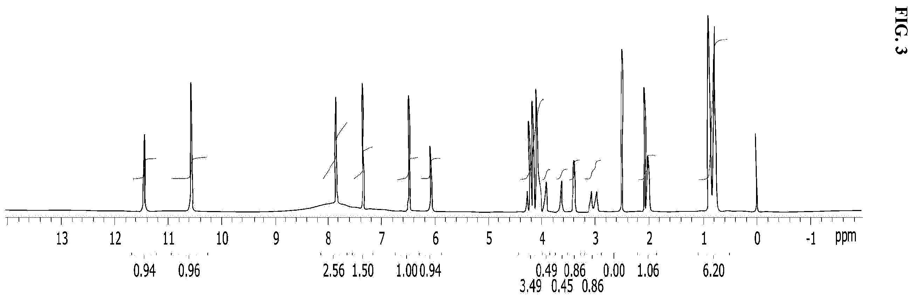

90 mL of methanol was added to 30 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, stirred, and heated up to 50°C. Then, 1.05 eq. of phosphoric acid was added thereto and stirred at 50°C for one hour to obtain a solution. The solution was cooled to room temperature, filtered, washed with methanol, and vacuum-dried to obtain the title compound (amount obtained 40 g, yield 85%) as an orange-colored powder. NMR analysis was performed to confirm the formation of the title compound. The results thereof were shown in FIG. 3.

### Example 4: Preparation of camsylate salt

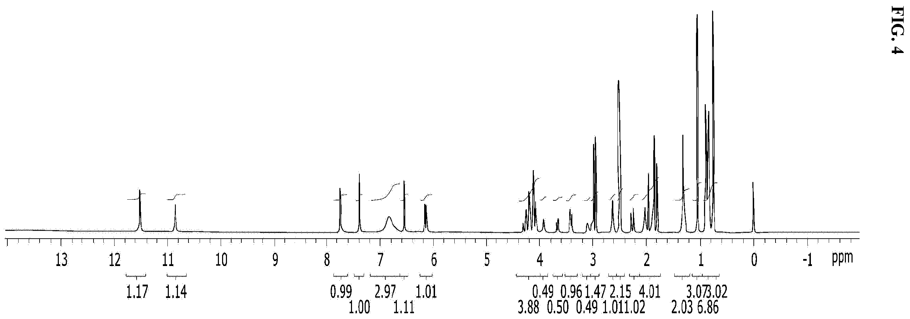

200 mL of ethanol was added to 20 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, and stirred. Then, 1.05 eq. of camphorsulfonic acid was added thereto, stirred at room temperature, filtered, washed with ethanol, and vacuum-dried to obtain the title compound (amount obtained 24.1 g, yield 92%) as a yellow powder. NMR analysis was performed to confirm the formation of the title compound. The results thereof were shown in FIG. 4.

### Example 5: Preparation of oxalate salt

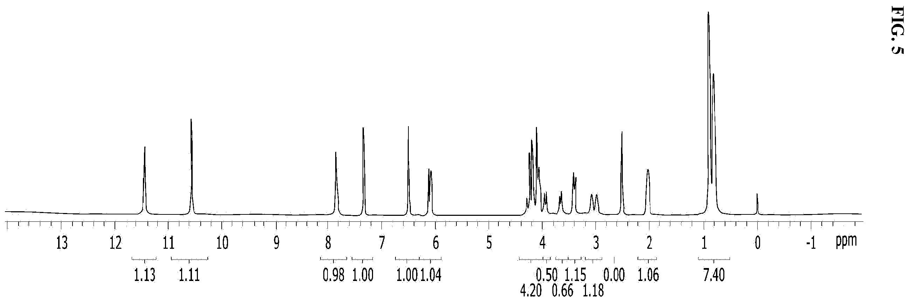

510 mL of acetonitrile was added to 30 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, and stirred. Then, 1.05 eq. of oxalic acid was added thereto, stirred at room temperature, filtered, washed with acetonitrile, and vacuum-dried to obtain the title compound (amount obtained 29.1 g, yield 66%) as a yellow powder. NMR analysis was performed to confirm the formation of the title compound. The results thereof were shown in FIG. 5.

### Example 6: Preparation of mesylate salt

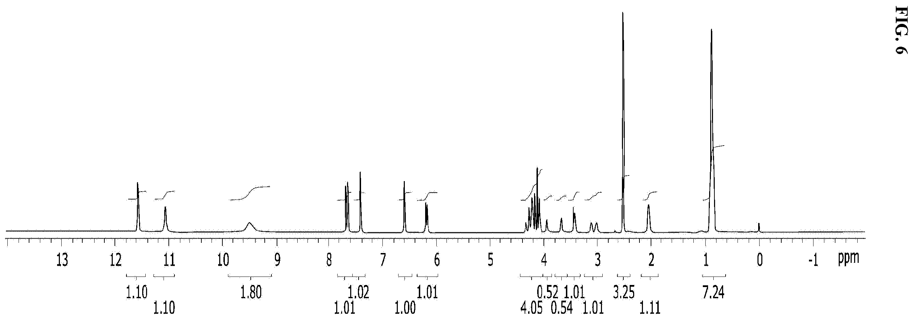

300 mL of ethyl acetate was added to 30 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, stirred, and heated up to 50°C. Then, 1.1 e.q. of methanesulfonic acid was slowly added dropwise thereto, continuously stirred at the same temperature, filtered, washed with methanol, and vacuum-dried to obtain the title compound (amount obtained 35.3 g, yield 93%) as an orange-colored powder. NMR analysis was performed to confirm the formation of the title compound. The results thereof were shown in FIG. 6.

### Example 7: Preparation of napadisylate salt

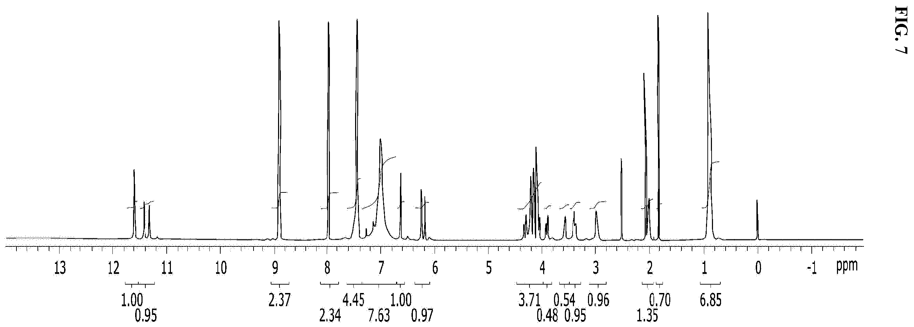

20 mL of acetonitrile was added to 1 g of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, and stirred. Then, a temperature was raised to 50°C, and 1.05 eq. of 1,5-naphthalenedisulfonic acid was added thereto, after which the resulting mixture was stirred, filtered, washed, and vacuum-dried to obtain the title compound. NMR analysis was performed to confirm the formation of the title compound. The results thereof were shown in FIG. 7.

### Analysis and measurement method

### 1. Measurement of water solubility

The water solubility was measured by shaking a saturated solution (20 mg/0.5 mL) at room temperature for 24 hours. A liquid phase was taken out of the solution and filtered using a 0.22 µm PVDF filter. The filtered liquid phase was diluted 10-fold for LC analysis.

LC analysis was performed by the following method.

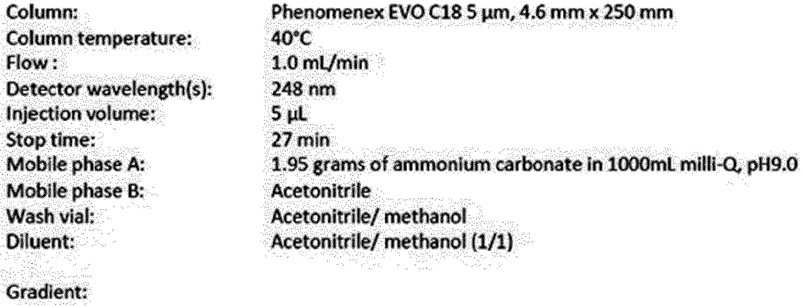

| **Time** *(min)* | **Flow** *(ml*/*min)* | **Mobile phase *(v*/*v%)*** | |
|---|---|---|---|
| | | **A** | **B** |
| 0 | 1.0 | 80 | 20 |
| 15 | 1.0 | 55 | 45 |
| 20 | 1.0 | 55 | 45 |
| 20.1 | 1.0 | 80 | 20 |
| 27 | 1.0 | 80 | 20 |

### 2. Physical stability evaluation experiment

In order to evaluate physical stability, samples were packaged in the form of ((LPDE+N2)+silica gel 1 g+LDPE)+Al-Bag, and stored under stress conditions (60°C±2°C/80% RH±5%) for 2/4 weeks before evaluation.

### 3. Photostability evaluation experiment

Samples were packaged in LPDE bag, and stored under visible (1.2M Lux-h), UV (200 W-h/m²) and UV+visible conditions, respectively, and then evaluated.

### 4. Hygroscopicity evaluation experiment

Samples were placed in a glass desiccator in which relative humidity was adjusted to 33%, 53%, 75%, and 93%, and then stored for two and four weeks to be evaluated.

### Analysis/measurement/evaluation results

### 1. Results of water solubility measurement

The results of measuring the water solubility of the salts obtained according to Examples 1 to 6 are shown in Table 1 below.

**[Table 1]**

| Classification | Water solubility (mg/mL) |
|---|---|
| Example 1 (hydrochloride salt) | 5.9 |
| Example 2 (hydrobromide salt) | 5.9 |
| Example 3 (phosphate salt) | 3.9 |
| Example 4 (camsylate salt) | 1.9 |
| Example 5 (oxalate salt) | 4.6 |
| Example 6 (mesylate salt) | 0.9 |

Referring to table 1, it can be confirmed that the water solubility of the salts according to the present invention is at least 0.9 mg/mL. In particular, it can be confirmed that the water solubility of hydrochloride salt, hydrobromide salt, camsylate salt, oxalate salt and phosphate salt is excellent at 1.9 mg/mL or more, and moreover, the hydrochloride salt, hydrobromide salt and oxalate salt exhibit very excellent water solubility of more than 4 mg/mL.

### 2. Physical stability evaluation results

The results of evaluating the physical stability of the salts obtained according to Examples 1 to 4 are shown in table 2 below.

In table 2, initial is a result measured after preparation of salts, and S2W and S4W are results measured after two and four weeks while being exposed to stress conditions, respectively.

**[Table 2]**

| **Example 1 (hydrochloride salt)** | | | | |
|---|---|---|---|---|
| Item | Criteria | Initial | S2W | S4W |
| Purity | Individual related material: 0.5% or less | 0.13 | 0.13 | 0.34 |
| | Total related material: 3.0% or less | 0.66 | 1.08 | 1.28 |
| Content | 97.0 ~ 102.0 % (anhydride, as anhydride) | 97.9 | 98.0 | 98.6 |
| C.I quantitative | 8.2 ~ 10.0 % (as anhydride 90-110%) | 9.2 | 9.1 | 9.1 |
| Moisture | Note | 3.33 | | - |

| **Example 2 (hydrobromide salt)** | | | | |
|---|---|---|---|---|
| Item | Criteria | Initial | S2W | S4W |
| Purity | Individual related material: 0.5% or less | 0.12 | 0.11 | 0.14 |
| | Total related material: 3.0% or less | 0.56 | 0.63 | 0.58 |
| Content | 97.0 ~ 102.0 % (anhydride, as anhydride) | 100.7 | 101.4 | 99.2 |
| C.I quantitative | 16.4 ~ 20.0 % (as anhydride 90-110%) | 18.1 | 17.8 | 17.8 |
| Moisture | Note | 3.93 | 3.93 | 3.79 |

| **Example 3 (phosphate salt)** | | | | |
|---|---|---|---|---|
| Item | Criteria | Initial | S2W | S4W |
| Purity | Individual related material: 0.5% or less | 0.08 | 0.08 | 0.11 |
| | Total related material: 3.0% or less | 0.41 | 0.43 | 0.53 |
| Content | 97.0 ~ 102.0 % (anhydride, as anhydride) | 98.9 | 99.9 | 99.9 |
| C.I quantitative | 19.1 ~ 25.5 % (as anhydride 90-110%) | 24.0 | 24.1 | 23.8 |
| Moisture | Note | 0.2 | 0.2 | 0.2 |

| **Example 4 (camsylate salt)** | | | | |
|---|---|---|---|---|
| Item | Criteria | Initial | S2W | S4W |
| Purity | Individual related material: 0.5% or less | 0.13 | 0.23 | 0.30 |
| | Total related material: 3.0% or less | 1.13 | 1.32 | 1.31 |
| Content | 97.0 ~ 102.0 % (anhydride, as anhydride) | 100.4 | 101.9 | 101.9 |
| C.I quantitative | 35.1 ~ 42.9 % (as anhydride 90-110%) | 40.3 | 40.3 | 40.6 |
| Moisture | Note | 0.67 | | 0.98 |

Referring to table 2, it can be confirmed that the salts according to the present invention have excellent stability against moisture and heat through the results of purity, content, etc., before/after exposure to stress conditions. In particular, in terms of purity, it could be confirmed that even after exposure to stress conditions, each of individual related materials and total related materials meets the quality standards of the raw material medicines.

### 3. Photostability evaluation results

The results of the salts according to Examples 1 to 5 of the present invention obtained according to the method of evaluating photostability are shown in table 3 below.

**[Table 3]**

| | | **Example 1 (hydrochloride salt)** | | **Example 2 (hydrobromide salt)** | | **Example 3 (phosphate salt)** | |
|---|---|---|---|---|---|---|---|
| | | Individual related material | Total related material | Individual related material | Total related material | Individual related material | Total related material |
| **Initial** | | 0.13 | 0.66 | 0.12 | 0.56 | 0.08 | 0.45 |
| **Solid** | **Control** | 0.11 | 0.55 | 0.11 | 0.44 | 0.07 | 0.37 |
| | **UV** | 0.36 | 2.31 | 0.11 | 0.60 | 0.10 | 0.42 |
| | **Vis** | 0.66 | 3.83 | 0.11 | 0.69 | 0.07 | 0.41 |
| | **UV+Vis Control** | 0.12 | 0.65 | 0.12 | 0.48 | 0.08 | 0.48 |
| | **UV+Vis** | 0.81 | 4.74 | 0.12 | 0.91 | 0.08 | 0.50 |

| | | **Example 4 (camsylate salt)** | | **Example 5 (oxalate salt)** | | | |
|---|---|---|---|---|---|---|---|
| | | Individual related material | Total related material | Individual related material | Total related material | | |
| **Initial** | | 0.13 | 1.13 | 0.14 | 0.64 | | |
| **solid** | **Control** | 0.14 | 0.14 | 0.65 | 0.65 | | |
| | **UV** | 0.13 | 0.27 | 1.00 | 1.00 | | |
| | **Vis** | 0.13 | 0.68 | 1.72 | 1.72 | | |
| | **UV+Vis Control** | 0.14 | 0.14 | 0.78 | 0.78 | | |
| | **UV+Vis** | 0.13 | 0.72 | 1.96 | 1.96 | | |

Referring to table 3, it can be confirmed that the salts according to the present invention have excellent stability against ultraviolet rays and visible light. In particular, in terms of purity, it could be confirmed that even after exposure to accelerated conditions, each of individual related materials and total related materials meets the quality standards of the raw material medicines.

### 4. Hygroscopicity evaluation results

The results of the salts according to Examples 1 to 4 of the present invention obtained according to the method of evaluating hygroscopicity are shown in table 4 below.

**[Table 4]**

| **Example 1 (hydrochloride salt)** | | | | | | |
|---|---|---|---|---|---|---|
| **Humidity** | **Individual related material** | | **Total related material** | | **Water(%)** | |
| | **2W** | **4W** | **2W** | **4W** | **2W** | **4W** |
| **Initial** | 0.13 | 0.13 | 0.66 | 0.66 | 3.33 | 3.33 |
| **33** | 0.12 | 0.11 | 0.68 | 0.62 | 5.22 | 5.32 |
| **53** | 0.12 | 0.12 | 0.78 | 0.78 | 5.21 | 5.38 |
| **75** | 0.12 | 0.11 | 0.69 | 0.68 | 5.24 | 5.41 |
| **93** | 0.12 | 0.13 | 0.89 | 0.94 | 5.37 | 5.43 |

| **Example 2 (hydrobromide salt)** | | | | | | |
|---|---|---|---|---|---|---|
| **Humidity** | **Individual related material** | | **Total related material** | | **Water(%)** | |
| | **2W** | **4W** | **2W** | **4W** | **2W** | **4W** |
| **Initial** | 0.12 | 0.12 | 0.56 | 0.56 | 3.93 | 3.93 |
| **33** | 0.11 | 0.10 | 0.53 | 0.55 | 3.70 | 3.94 |
| **53** | 0.12 | 0.11 | 0.66 | 0.59 | 3.62 | 3.96 |
| **75** | 0.11 | 0.11 | 0.58 | 0.61 | 3.78 | 3.93 |
| **93** | 0.11 | 0.10 | 0.54 | 0.62 | 3.71 | 3.94 |

| **Example 3 (phosphate salt)** | | | | | | |
|---|---|---|---|---|---|---|
| **Humidity** | **Individual related material** | | **Total related material** | | **Water(%)** | |
| | **2W** | **4W** | **2W** | **4W** | **2W** | **4W** |
| **Initial** | 0.07 | 0.07 | 0.37 | 0.37 | 0.61 | 0.61 |
| **33** | 0.07 | 0.08 | 0.43 | 0.41 | 0.41 | 0.53 |
| **53** | 0.08 | 0.07 | 0.43 | 0.35 | 0.53 | 0.66 |
| **75** | 0.08 | 0.07 | 0.44 | 0.39 | 0.60 | 0.81 |
| **93** | 0.08 | 0.07 | 0.41 | 0.33 | 1.17 | 0.97 |

| **Example 4 (camsylate salt)** | | | | | | |
|---|---|---|---|---|---|---|
| **Humidity** | **Individual related material** | | **Total related material** | | **Water(%)** | |
| | **2W** | **4W** | **2W** | **4W** | **2W** | **4W** |
| **Initial** | 0.13 | 0.13 | 1.13 | 1.13 | 0.67 | 0.67 |
| **33** | 0.14 | 0.14 | 1.10 | 1.07 | 0.56 | 0.54 |
| **53** | 0.15 | 0.15 | 1.18 | 1.04 | 0.80 | 0.65 |
| **75** | 0.15 | 0.16 | 1.18 | 1.18 | 1.44 | 1.02 |
| **93** | 0.16 | 0.17 | 1.17 | 1.15 | 2.03 | 1.72 |

Referring to table 4, it can be confirmed that the salts according to the present invention have low hygroscopicity.

### Experimental Example 1 - ADP-Glo^{™} kinase assay

### (1) JAK1 kinase assay

10 mM of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide (hereinafter, referred to as Compound 1), which is the heterocyclic compound represented by formula II obtained according to Preparation Example 1, was diluted in DMSO to prepare samples at five concentrations (1,000 nM, 200 nM, 40 nM, 8 nM, and 0.16 nM). 10 mM of ATP and 10 mg/mL of insulin receptor substrate (IRS) were diluted at 1/40 and 1/50, respectively, in 1X kinase buffer. In addition, 222 ng/µL of JAK1 enzyme was diluted at 1/8 in 1X kinase buffer to make a preparation. 1 µL of ATP, 1 µL of IRS, and 1 µL of Compound 1 prepared as above were mixed, after which 2 µL of JAK1 enzyme was finally added and reacted at 30°C for 40 minutes. At this time, a tube without JAK1 enzyme was prepared for a blank and a tube without Compound 1 was prepared as a positive control group together (a final concentration of samples was 200 nM, 40 nM, 8 nM, 1.6 nM, and 0.32 nM).

Then, 5 µL of ADP-glo was added to each tube and reacted at 30°C for 40 minutes, after which 10 µL of kinase detection reagent was added thereto and reacted at room temperature for 15 minutes. After 18 µL of the reactant was transferred to a 384 well plate, luminescence was measured using a plate reader.

### (2) JAK2 kinase assay

10 mM of Compound 1 was diluted in DMSO to prepare samples at four concentrations (5,000 nM, 500 nM, 50 nM and 5 nM). 10 mM of ATP was diluted at 1/40 in 1X kinase buffer to make a preparation. 1 mg/mL of IGF1Rtide was used as a stock solution. 100 ng/µL of JAK2 enzyme was diluted at 1/20 in 1X kinase buffer to make a preparation. 1 µL of ATP, 1 µL of IGF1Rtide, and 1 µL of Compound 1 prepared as above were mixed, after which 2 µL of JAK2 enzyme was finally added and reacted at 30°C for 40 minutes. At this time, a tube without JAK2 enzyme was prepared as a blank and a tube without Compound 1 was prepared as a positive control group together (a final concentration of samples was 1,000 nM, 100 nM, 10 nM, and 1 nM).

Then, 5 µL of ADP-glo was added to each tube and reacted at 30°C for 40 minutes, after which 10 µL of kinase detection reagent was added thereto and reacted at room temperature for 15 minutes. After 18 µL of the reactant was transferred to a 384 well plate, luminescence was measured using a plate reader.

### (3) JAK3 kinase assay

10 mM of Compound 1 was diluted in DMSO to prepare samples at four concentrations (50,000 nM, 5,000 nM, 500 nM and 50 nM). 10 mM of ATP was diluted at 1/40 in 1X kinase buffer to make a preparation. 1 mg/mL of Poly (Glu₄, Tyr₁) peptide was used as a stock solution. 100 ng/µL of JAK3 enzyme was diluted at 1/20 in 1X kinase buffer to make a preparation. 1 µL of ATP, 1 µL of Poly (Glu₄, Tyr₁) peptide, and 1 µL of Compound 1 prepared as above were mixed, after which 2 µL of JAK3 enzyme was finally added and reacted at 30°C for 40 minutes. At this time, a tube without JAK3 enzyme was prepared as a blank and a tube without Compound 1 was prepared as a positive control group together (a final concentration of samples was 10,000 nM, 1,000 nM, 100 nM, and 10 nM).

Then, 5 µL of ADP-glo was added to each tube and reacted at 30°C for 40 minutes, after which 10 µL of kinase detection reagent was added thereto and reacted at room temperature for 15 minutes. After 18 µL of the reactant was transferred to a 384 well plate, luminescence was measured using a plate reader.

### (4) Tyk2 kinase assay

10 mM of Compound 1 was diluted in DMSO to prepare samples at five concentrations (5,000 nM, 500 nM, 50 nM, 5 nM, and 0.5 nM). 10 mM of ATP was diluted at 1/40 in 1X kinase buffer to make a preparation. 1 mg/mL of Poly (Glu₄, Tyr₁) peptide was used as a stock solution. 100 ng/µL of Tyk2 enzyme was diluted at 1/2 in 1X kinase buffer to make a preparation. 1 µL of ATP, 1 µL of Poly (Glu₄, Tyr₁) peptide, and 1 µL of Compound 1 prepared as above were mixed, after which 2 µL of Tyk2 enzyme was finally added and reacted at 30°C for 40 minutes. At this time, a tube without JAK3 enzyme was prepared for a blank and a tube without Compound 1 was prepared as a positive control group together (a final concentration of samples was 1,000 nM, 100 nM, 10 nM, 1 nM, and 0.1 nM).

Then, 5 µL of ADP-glo was added to each tube and reacted at 30°C for 40 minutes, after which 10 µL of kinase detection reagent was added thereto and reacted at room temperature for 15 minutes. After 18 µL of the reactant was transferred to a 384 well plate, luminescence was measured using a plate reader.

### Experimental Example 2 - In vitro cell assay

### 1. Preparation of experimental cell (cell line)

UT-7/EPO cell is an erythroleukemic cell line which expresses an EPO receptor, and is frequently used in many previous studies as a model to assess EPO-induced STAT5 phosphorylation mediated by JAK1 and JAK2 activity. Accordingly, in order to evaluate the activity of JAK1 and JAK2, UT-7/EPO cells were prepared.

In addition, NK-92 cell is a natural killer cell line, which grows in an IL-2-dependent way, and is derived from peripheral blood mononuclear cells, and thus is suitable for evaluating the activity of JAK1 and JAK3 by IL-2. Thus, in order to evaluate the activity of JAK1 and JAK3, NK-92 cells were prepared.

Furthermore, there is a literature in which U20S cells are associated with JAK1/TYK2 signaling activity and phosphorylation of STAT1 by IFN-alphaB2 and IFN-γ. Accordingly, U2OS cells were prepared for the evaluation of JAK1 and TYK2 as a cell line which strongly expresses STAT1 signals.

### 2. Preparation of test material and stimulus source

### (1) Preparation of test material

The test material was dissolved in DMSO, prepared at 10 mM, divided by 20 µL each, and stored at -20°C.

### (2) Preparation of stimulus source

**EPO:** 2000 U/0.5 mL of stock was divided by 50 µL each and stored under refrigeration.

**IL-2**: 200 µg/ml of stock was prepared, divided by 5 µL each, and stored, and then treated at 2 ng/ml. (IL-2 1µg/ml=13000 U/ml)
**IFN-alphaB2:** 1 x 10 U/ml of stock was diluted and used, and then treated to reach 30000 U/ml.

### (3) Preparation of assay medium

**UT-7/EPO cell assay:** MEM-alpha (Gibco, cat # 12561-056) was prepared to contain 5% FBS and 1% P/S before use.

**NK-92 cell assay:** A preparation was made to contain HBSS, 1% FBS and 1% P/S before use.

**U2OS cell assay:** A preparation was made to contain HBSS, 1% FBS and 1% P/S before use.

### 3. Preparation of sample

The cultured cells were centrifuged (1,000 rpm, 5 minutes) and washed with PBS. The size of cell lines and appropriate reaction concentration thereof were set and the cell lines were seeded in a 96-well plate at 1 x 10⁵ to 10⁶ cells/well (50 µL/well). The test material was prepared 4X of the concentration to be treated, and prepared at five to six concentrations by serial dilution of 1/5 each. The test material was diluted 1: 1000 in assay medium and treated at 20 µL/well. An untreated group without drug treatment was treated with 20 µL/well of assay medium and incubated in a CO₂ incubator for one hour.

After one to two hours of treatment with the test material, cytokine was treated at 10 µL/well (final EPO concentration: 1 U/mL, final IL-2 concentration: 2 ng/mL, final IFN-alphaB2 concentration: 30000 U/mL). In this case, the test was designed to include a cytokine-only treatment group and a non-treatment group, and the non-treatment group was treated with 10 µL of assay medium only after cell seeding.

After stimulation with cytokine for 20 minutes as above, the cells were lysed in 5X RIPA buffer for 30 minutes and centrifuged at 13000 rpm for five minutes at 4°C. The supernatant was transferred to a new tube and subjected to ELISA or stored at -80°C until use.

### 4. ELISA performance

An ELISA was performed according to a protocol of cell signaling ELISA KIT (Cat no. 7113, 7234C).

### Experimental example 3 - Human whole blood assay

### 1. Evaluation of IL-6-induced STAT1 phosphorylation in TEST 1 - CD4⁺ cell

A stock solution of Compound 1 obtained according to Preparation Example 1 was diluted with distilled water and then serially diluted at 1/5 with 4% DMSO. 5 µL of test material was added to 100 µL of whole blood collected using a 1.7 mL eppendorf tube, mixed, and incubated at 37°C for 45 minutes.

IL-6 (10 µg/ml) was diluted with 0.1% BSA/DW to reach 1 µg/mL each. 5 µL of IL-6 was added (at a final concentration of 50 ng/mL) and incubated at 37°C for 15 minutes. At this time, 5 µL of DPBS was added as a non-stimulated control group. Lyse/fix buffer 5X was diluted 1X in distilled water and used.

After adding 900 µL of lyse/fix buffer, which was pre-warmed at 37°C, to each tube and incubating at 37°C for 20 minutes, centrifugation was performed at 500 xg for eight minutes in a centrifuge, after which the supernatant was removed and washed with 1 mL of FACS buffer. The washing process was repeated. Then, 400 µL of BD Phosflow^{™} Perm buffer, which was prestored in ice, was added thereto, incubated in ice for 30 minutes, and subjected into spin-down. After washing with a wash buffer once, the resulting product was resuspended with a buffer solution (BD Pharmingen^{™} staining buffer).

For double staining of CD4 and pSTAT1, anti-CD and anti-pSTAT1 were mixed in a staining buffer and added to each of the samples. The tube containing the sample was lightly hit by hand to mix the antibody and cells well and left overnight at 4°C. On the next day, the results were analyzed using FACSCanto II.

### 2. TESTs 2 to 6

Each of TESTs 2 to 6 was performed in substantially the same manner as experimented in TEST 1, except that the type and amount of cytokines were as shown in Table 5 below.

**[Table 5]**

| **Test no.** | **Test 1** | **Test 2** | **Test 3** | **Test 4** | **Test 5** | **Test 6** |
|---|---|---|---|---|---|---|
| **Cell marker** | CD4⁺ | CD14⁺CD33⁺ | CD4⁺ | CD4⁺ | CD3⁺CD56⁺ | Lih⁻CD34⁺ |
| **Trigger** | IL-6 50 ng/mi | GM-CSF 20 pg/rnl | IFN-α 1x10³ U/ml | IL-2 5 ng/ml | IL-23 500 ng/ml | EPO 10 U/ml |
| **Read-out** | pSTAT1 | pSTAT5 | pSTAT3 | pSTATS | pSTAT3 | pSTAT5 |

### 3. Analysis method - Data collection and IC₅₀ calculation

The numerical values analyzed by FlowJo were calculated to obtain IC₅₀ values using GraphPad Prism 5 software (product name).

No treatment response (NTR) was set as a reference, and the degree of the phosphorylation of STAT induced by cytokines was converted into a relative ratio (% control group). A dose-response plot was obtained for each concentration-dependent activity of each test material using GraphPad Prism (version 5.0) and IC₅₀ values were calculated.

### Results of Experimental Examples 1 to 3

The results obtained according to Experimental Examples 1 to 3 described above are as follows.

| Assay | IC₅₀ (nM) | | | | |
|---|---|---|---|---|---|
| In vitro enzyme | JAK1 | JAK2 | JAK3 | TYK2 | |
| | 3.8 | 68 | 315 | 22 | |
| Cell Biology | JAK1,2 | JAK2 | JAK1,3 | JAK1, Tyk2 | |
| | 18 | 656 | 163 | 27 | |
| Human whole blood | JAK1 | JAK2 | JAK1,Tyk2 | JAK1, 3 | JAK2, Tyk2 |
| | 70 | 774 | 168 | 544 | 3641 |

Referring to the results, it can be confirmed that Compound 1 according to the present invention has very excellent JAK1 inhibitory activity.

### Experimental Example 4 - IL-4 secretion inhibitory activity in basophil cells

RBL-2H3 cells were diluted in 10% FBS EMEM medium and divided in a 24-well plate to reach 1 X 105 cells/well. The cells were incubated in a 37°C incubator for 18 hours or longer.

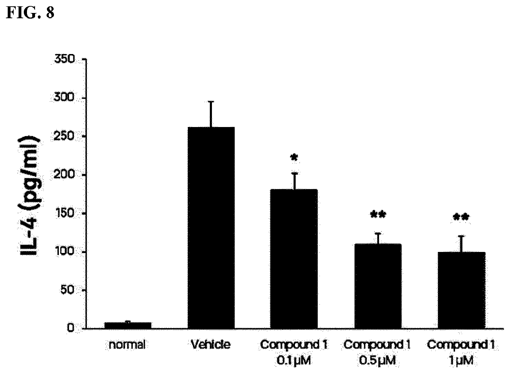

The test material (Compound 1) was treated at each concentration (at a final concentration of 0, 0.1, 0.5 and 1 µM) under PMA (50 nmol/L) and A23187 (1 µmol/L) stimulation. The cells were incubated in a 37°C incubator for 24 hours. Then, the reaction was terminated on ice, after the degree of IL-4 secretion in the culture supernatant was measured by IL-4 ELISA kit. The results thereof were shown in FIG. 8.

FIG. 8 is a graph showing the results of an experiment on IL-4 secretion inhibitory activity in Basophil cells with regard to Compound 1.

Referring to FIG. 8, it can be confirmed that the concentration of IL-4 is lower than that of the control group (vehicle) with regard to the groups treated with Compound 1 according to the present invention. In other words, it was confirmed that Compound 1 according to the present invention inhibits IL-4 secretion, and in particular, when Compound 1 is treated at a concentration of 0.5 µM and 1 µM, it can be remarkably reduced to almost 1/2 or more compared to the negative control group (vehicle).

### Experimental Example 5 - IL-13, IL-10 and TNF-α secretion activity

50 µL of DNase I (2000 unit/mL) was added to 50 mL of cell culture medium and mixed. (Since enzyme and cells were used, the medium was heated in a 37°C water bath before use.) In addition, PMBC of atopic patients was rapidly dissolved in a 37°C water bath.

PMBC was slowly flowed into the prepared medium, and reacted at 37°C for five minutes. Centrifugation was performed using a centrifuge at 200 rpm for 15 minutes, after which supernatants were removed, suspended in 1 mL of cell culture medium, and counted.

Cells were diluted in cell culture medium and seeded in a 96-well plate to reach 2 x 10⁵ cells/100 µL/well.

50 µL of PBMC activation medium (0.5 µg/ml CD3 antibody, 5 µg/ml CD28 antibody) was added to every well except the negative control well. (50 µL of cell culture medium was added to each negative control well.)

50 µL of the test material was added to each well according to each concentration. (final concentration of 1 nM, 10 nM, 100 nM, 1,000 nM, 50 µL of cell culture medium was added to each negative control well.)

After incubation in a 5% CO₂ incubator at 37°C for 24 hours, the plates were centrifuged at 200 rpm for 15 minutes as they were, and 150 µL of the suspension was taken, placed in a 96-well round bottom plate, and stored in a deep freezer at minus 80°C.

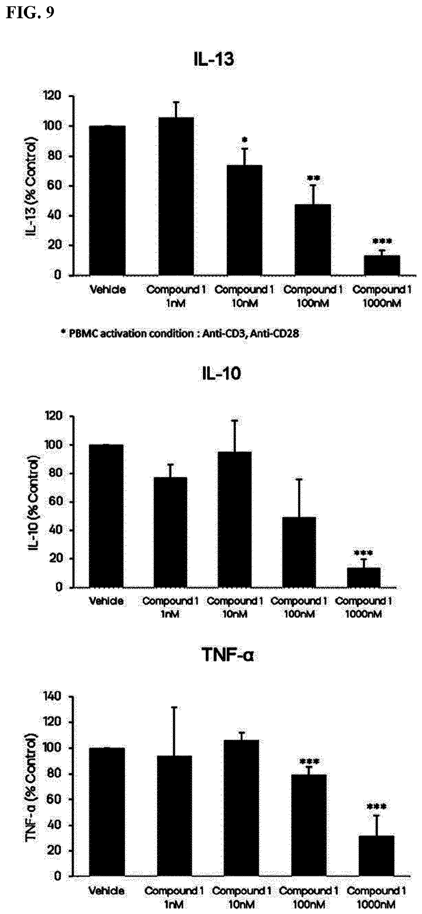

After the culture supernatant samples were dissolved, ELISA was performed according to the data sheet protocol of ELISA kit. The results thereof were shown in FIG. 9.

FIG. 9 is a view showing graphs of the evaluation results of IL-13, IL-10, and TNF-α secretion activity with regard to Compound 1 of the present invention.

Referring to FIG. 9, it can be confirmed that each of IL-13, IL-10 and TNF-α decreases in anti-CD3 and anti-CD28-activated PBMC of patients with atopic dermatitis. In particular, it can be confirmed that IL-13, IL-10 and TNF-α are significantly reduced in the groups in which Compound 1 is tested at a concentration of 100 nM and 1,000 nM, respectively.

### Experimental Example 6 - DNCB-induced model (topical)

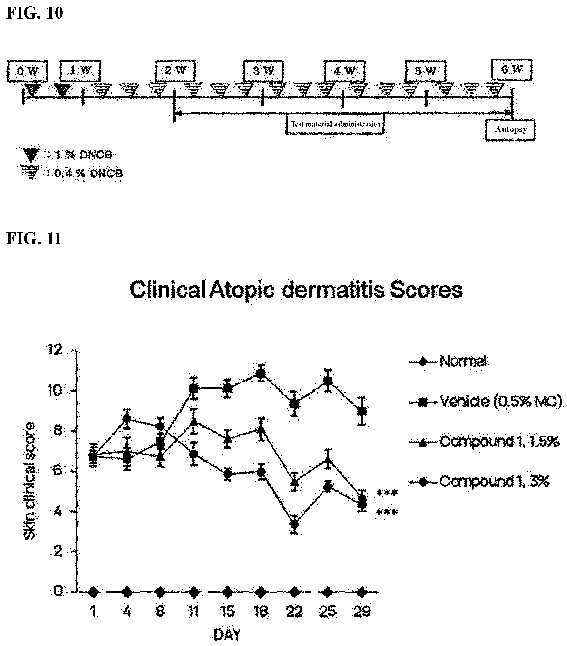

### 1. Induction of atopic model and treatment with test material (FIG. 10)

NC/Nga mice were anesthetized with isoflurane and the dorsal hair (from the bottom of the ear to the top of the tail) was then removed. The NC/Nga mice were left for 24 hours for recovery of fine wounds on the shaved skin.

After healing, 200 µL of 1% 1-chloro-2,4-dinitrobenzene (DNCB) in acetone/olive oil (3:1) was topically applied to the dorsal skin (primary skin application). Secondary skin application was performed after three days for sensitization.

Seven days after the primary skin application, 150 µL of 0.4% DNCB in acetone/olive oil (3:1) was topically applied, and applied to the dorsal skin three times a week for five weeks.

The phosphate of Compound 1 (hereinafter, referred to as a phosphate compound) obtained according to Example 3 was used as a test material, and the test material was treated before applying DNCB from two weeks after the primary skin application. The dosage of the test material was calculated based on a body weight of mice measured on the day of administration, and the test material was topically applied to atopic skin using a glass stick (a total of 56 times twice a day for 28 days, six hours at a treatment interval). 0.5% methylcellulose (0.5% MC) was administered to the control group (vehicle).

### 2. Autopsy

Week 6 after the primary skin application, the test animals were autopsied. Specifically, the test animals were anesthetized using isoflurane, and as much blood as possible was drawn from the caudal vena cava with a syringe and stored in a 5 mL vacuum tube containing a blood clot activator. The blood was allowed to stand at room temperature for 15-20 minutes for coagulation and then centrifuged at 3000 rpm for 10 minutes.

### 3. Evaluation method

### (1) Clinical atopic dermatitis score

Atopic dermatitis scores were rated as none (0), mild (1), moderate (2), and severe (3) for each of the five symptoms (erythema, dryness, skin edema and hematoma, erosion, and keratinization). The total dermatitis score representing clinical severity was defined as the sum of all scores (maximum score: 15). The evaluation was conducted twice a week by the same researcher at the same time.

### (2) Itching behavior test

To evaluate a scratching behavior at week 4, mice were placed in cages and the frequency of scratching was evaluated for 30 minutes.

### (3) Serum IgE and histamine

Quantitative analysis was performed using a serum IgE and histamine with ELISA kit.

### (4) Spleen/weight

After measuring a weight of the spleen and mouse, a spleen-to-body weight ratio (relative organ weight) was calculated.

### (5) qRT-PCR

TARC, TSLP, IL-4, IL-1β and TNF-α were quantified using skin tissue through qRT-PCR.

### (6) Histopathological analysis

On the day of autopsy, the skin in 10% neutral buffered formalin solution was analyzed by staining with H & E and toluidine blue.

Referring to all the evaluation results of Experimental Example 6 in FIGS. 11 to 16, it can be confirmed that the phosphate compound according to the present invention ameliorates symptoms of atopic dermatitis. In FIGS. 11 to 16, "Compound 1" means the "phosphate compound" obtained according to Example 3.

Specifically, FIG. 11 is a graph showing the results of clinical atopic dermatitis score in Experimental Example 6, and referring to this, it can be confirmed that a skin severity score of a group dosed with the phosphate compound is significantly reduced.

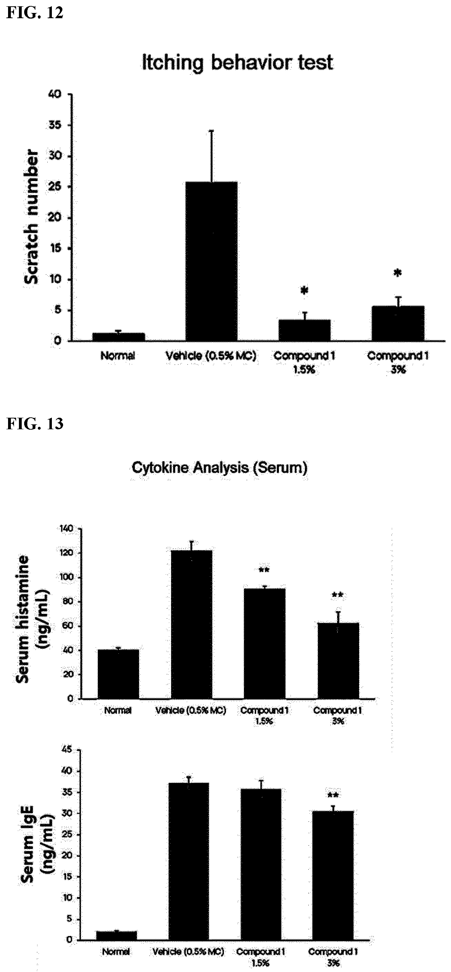

FIG. 12 is a graph showing the results of itching behavior test in Experimental Example 6. Referring to FIG. 12, it can be confirmed that a scratching behavior in the group dosed with the phosphate compound is reduced by at least 30% compared to the control group (vehicle). In particular, it can be confirmed that the scratching behavior in the group dosed with 3% phosphate compound is at least 50% compared to the control group (vehicle), which is significantly reduced to a normal mouse level.

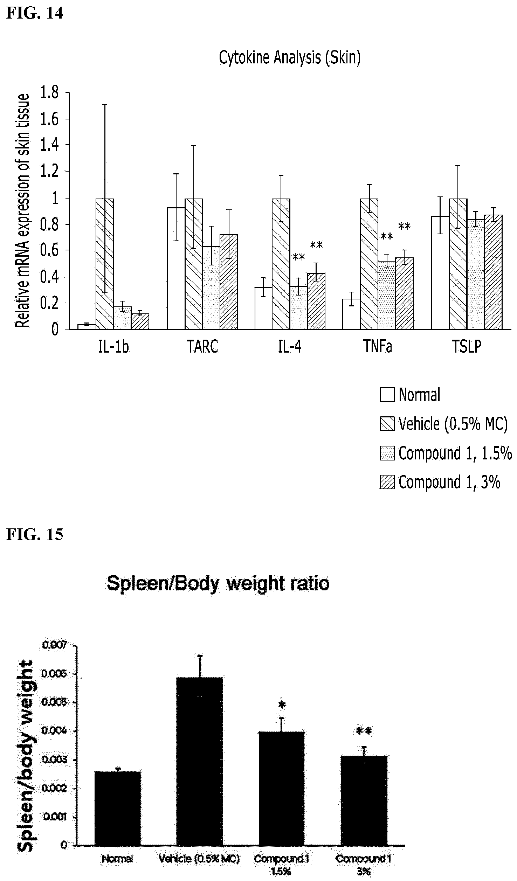

FIGS. 13 and 14 are views showing the results of cytokine analysis (plasma, skin) in Experimental Example 6, FIG. 13 (upper) is a graph showing a plasma histamine concentration (ng/mL), and FIG. 13 (lower) shows a plasma IgE concentration (ng/mL). Referring to FIGS. 13 and 14, it can be confirmed that atopic dermatitis-related cytokines of plasma and skin are reduced in the group dosed with the phosphate compound.

FIG. 15 is a graph showing the results of calculation of the spleen-to-body weight ratio in Experimental Example 6, and it can be confirmed that the spleen-to-body weight ratio value is decreased in the group dosed with the phosphate compound.

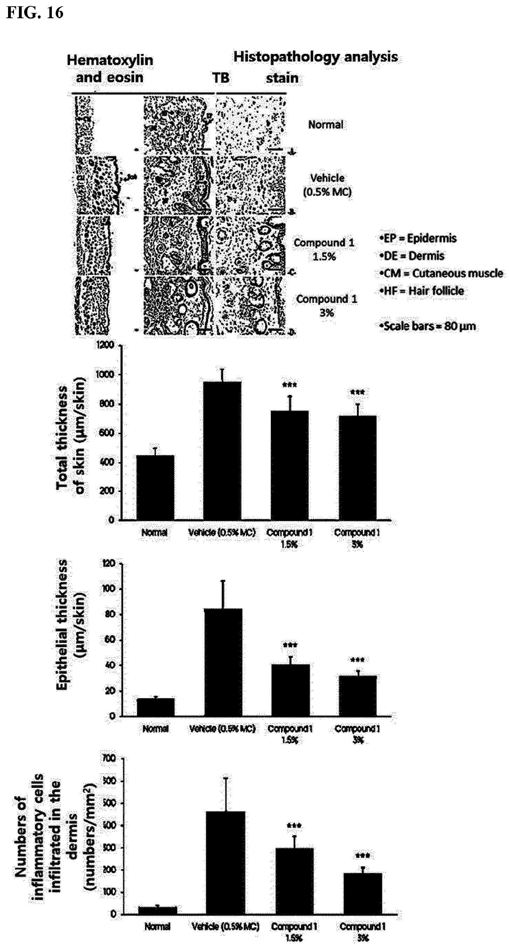

FIG. 16 is a view showing the results of skin staining in Experimental Example 6, and it can be confirmed that treatment with the phosphate compound changes the histological properties of atopic dermatitis.

### Experimental Example 7 - DNCB-induced model (PO)

A DNCB-induced animal model was prepared in substantially the same manner as the DNCB model (topical) in Experimental Example 6, except that the phosphate compound was orally administered, and the phosphate compound obtained according to Example 3 was administered. The phosphate compound was orally administered a total of 56 times twice a day for 28 days, and an administration interval was eight hours. The dosage (10 mL/kg) was calculated and determined based on the body weight measured on the day of administration, and intragastrically administered through the mouth of mice using a 1 mL syringe and a feeding needle. The control group was dosed with 0.5% methylcellulose (0.5% MC).

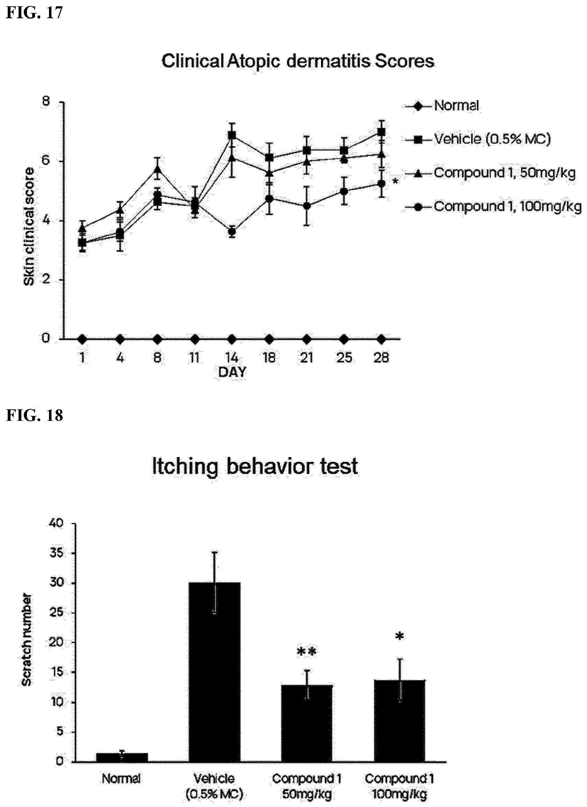

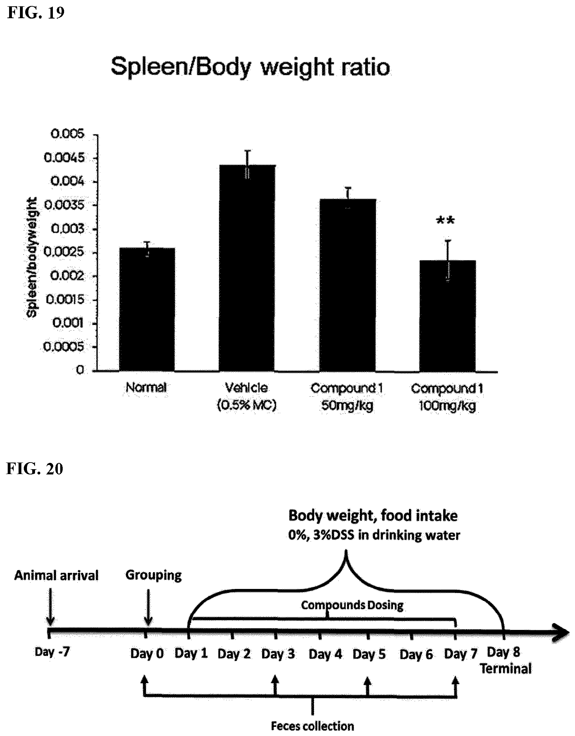

FIG. 17 is a graph showing the results of clinical atopic dermatitis score in Experimental Example 7, FIG. 18 is a graph showing the results of itching behavior test in Experimental Example 7, and FIG. 19 is a graph showing the results of calculation of the spleen-to-body weight ratio in Experimental Example 7. In FIGS. 17 to 19, "Compound 1" means the "phosphate compound" obtained according to Example 3.

Referring to all the evaluation results of Experimental Example 7 in FIGS. 17 to 19, it can be confirmed that the phosphate compound according to the present invention ameliorates symptoms of atopic dermatitis.

### Experimental Example 8 - DSS-induced model (FIG. 20)

### 1. Induction of colitis and treatment with test material

To induce colitis in C57BL/6J mice, drinking water was treated with 3% dextran sodium sulphate (DSS) and provided each morning for eight days, and a negative control group was provided with 0% DSS.

After mice were grouped into several groups (day O), the mice were orally dosed with 0.5% methylcellulose and Compound 1 obtained according to Preparation Example 1 as a test material at 9 a.m. and 5 p.m. every day from day 1 to day 7. Test animals were autopsied on day 8.

### 2. Analysis method

### (1) Animal's disease activity index

Disease activity index (DAI) was calculated as body weight, fecal concentration, and fecal blood score to represent the severity of the disease. Body weight and food intake were monitored and evaluated at the start of the study and every 24 hours, and fecal samples were collected on days 0, 3, 5, and 7 and analyzed according to Table 6 below.

**[Table 6]**

| **Score** | **Stool consistency** | **Occult blood,** |
|---|---|---|
| 0 | Normal | Negative hemoccult |
| 1 | Soft but still formed | Positive hemoccult |
| 2 | Very soft | Blood traces in stool visible |
| 3 | Diarrhea rectal | Bleeding |

### (2) Weight-to-length ratio of colon

After measuring the weight and length of the colon obtained by autopsy, the weight-to-length ratio was calculated.

### (3) Detection of biomarker in plasma and large intestine

On day 8, blood was drawn from the heart into a centrifuge tube containing EDTA-K2 anticoagulant. After centrifugation (7000 rpm, 10 minutes, 4°C), plasma was separated, and KC and MIP-2 were measured by Meso Scale Discovery Kit.

The mRNA was extracted from a portion of large intestine tissues (same location for each animal) to analyze an expression level of MX2 and TNF-alpha in the tissues through qPCR.

### (3) Histopathological score

The large intestine part fixed with 10% formalin was cut with paraffin and stained with hematoxylin-eosin. The criteria for histological changes in the colon followed Table 7 below.

**[Table 7]**

| Score | Histologic changes |
|---|---|
| 0 | No evidence of inflammation |
| 1 | Low level of inflammation with scattered infiltrating mononuclear cells (1-2 foci). |
| 2 | Moderate inflammation with multiple foci |
| 3 | High level of inflammation with increased vascular density and marked wall thickening |
| 4 | Maximal severity of inflammation with transmural leukocyte infiltration and loss of goblet cells |

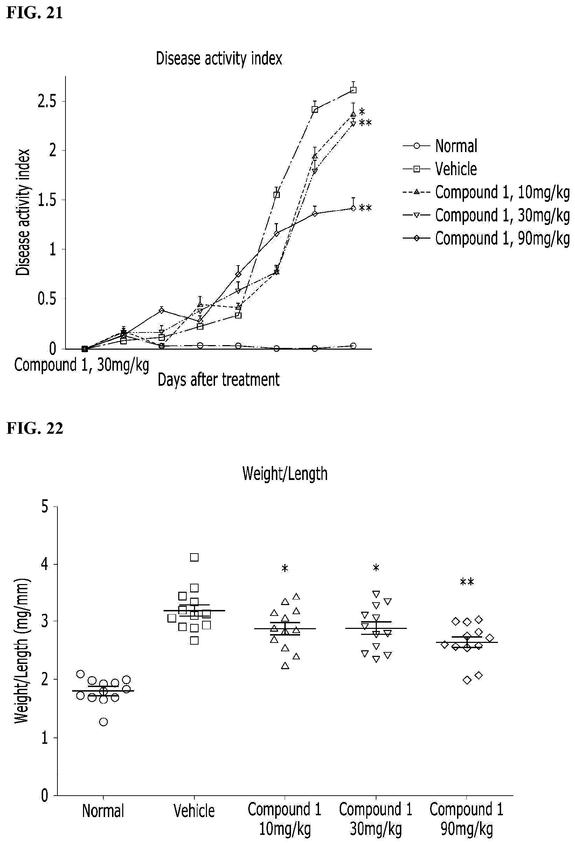

In FIGS. 21 to 24, "Compound 1" means Compound 1 (freebase) obtained according to Preparation Example 1.

FIG. 21 is a graph showing the results of analyzing disease activity index (DAI) in Experimental Example 8.

Referring to FIG. 21, it can be confirmed that the disease activity index increases over time in all groups except the normal group, but the degree of increase in the disease activity index of the groups dosed with Compound 1 of the present invention is lower than that of the control group (vehicle). In particular, it can be seen that in the group dosed with 90 mg/kg of Compound 1, the disease activity index is reduced to half that of the control group (vehicle).

FIG. 22 is a graph showing the results of the weight-to-length ratio of the colon in Experimental Example 8.

Referring to FIG. 22, it can be seen that the weight-to-length ratio is high in all groups compared to the normal group. However, the group dosed with Compound 1 has a lower value compared to the control group (vehicle). As a result, it can be confirmed that the weight of the large intestine increases and the length of the large intestine decreases in the control group as a condition with inflammation produced in the large intestine is maintained or worsened, whereas the colitis is ameliorated and the nature of disease is weakened in the groups dosed with Compound 1.

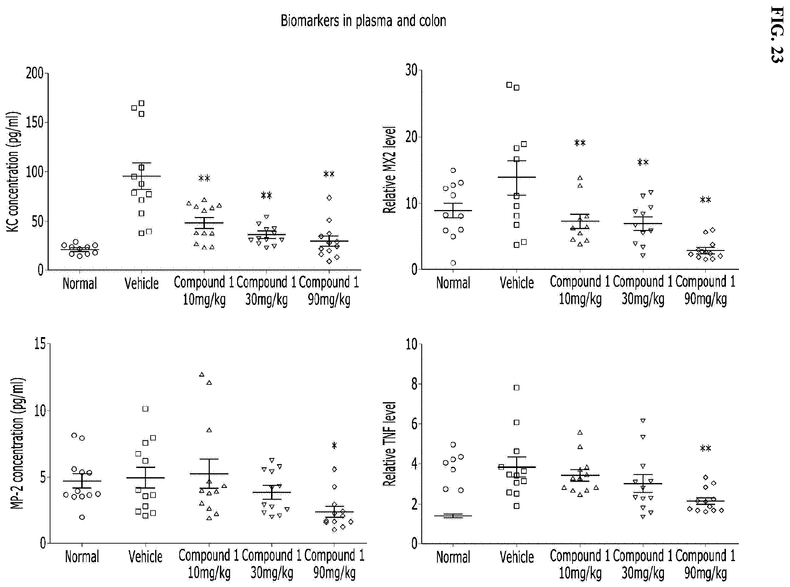

FIG. 23 is a view showing the results of detecting biomarkers in plasma and large intestine of Experimental Example 8.

Referring to FIG. 23, it can be confirmed that each concentration of KC and MIP-2, which are biomarkers increased in plasma in the colitis environment, is decreased in the groups dosed with Compound 1 of the present invention. In addition, it can be confirmed that an expression level of MX2 and TNF-alpha is also decreased in the colon of the groups dosed with Compound 1 of the present invention. As a result, it can be seen that an increase in biomarkers related to inflammation in the colon is also prevented.

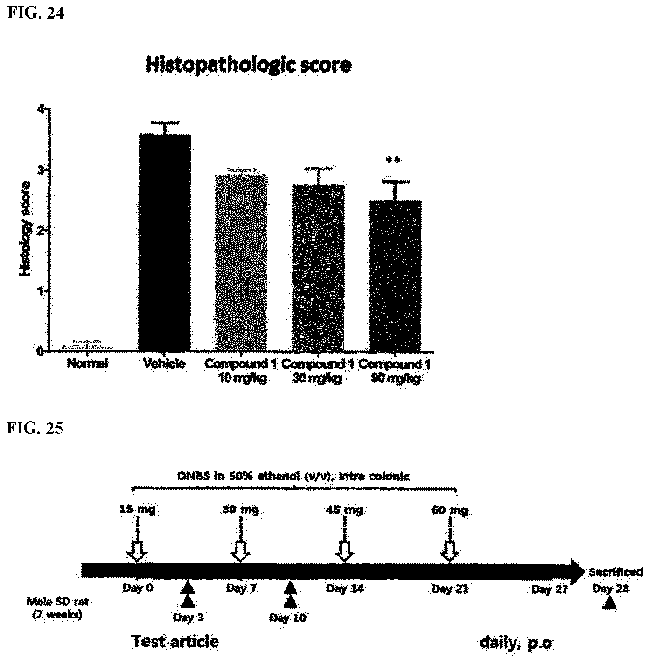

FIG. 24 is a graph showing the results of histopathological score in Experimental Example 8, and it can be confirmed that a histopathological score is lowered in the groups dosed with Compound 1 of the present invention.

### Experimental Example 9 - DNBS-induced model (FIG. 25)

### 1. Induction of colitis and treatment with test material

Before induction of colitis with DNBS every week, experimental animals were fasted for 24 hours. Fasted animals were lightly anesthetized with isoflurane, catheter tubes with a length of 8 cm were inserted into the rat anus, and then colitis was induced by slowly injecting 250 µL of DNBS [15 mg (1st week), 30 mg (2nd week), 45 mg (3rd week) and 60 mg (4th week) in 250 µL of 50% ethanol] solution using a syringe pump at a rate of 100 µL/min. After the injection of the solution, a head-down posture was maintained for one minute so that the solution may spread evenly in the animal's intestines and may not leak out of the body.

Using Compound 1 obtained according to Preparation Example 1 as a test material, the test material was converted into a dosage for each individual based on the body weight and then orally administered at 10 mL per kg of body weight once a day for seven days a week, a total of four weeks.

### 2. Evaluation method

### (1) Measurement of weight-to-length ratio of large intestine

The large intestine tissue extracted from the sacrificed experimental animals was photographed to measure the length of the large intestine. The length of the large intestine was measured from the taken picture using an image program (Leica Application Suite V4). After taking the picture, the tissue was opened around a longitudinal axis after removing the cecum, washed with physiological saline, and weighed. The measured weight and length were used to convert the large intestine ratio (mg/cm) (= large intestine weight (mg)/large intestine length (cm)).

### (2) Measurement of biomarker in serum

Calprotectin & c-reactive protein (CRP) and IFN-γ from serum collected at autopsy were analyzed by ELISA.

### (3) Immunohistochemical staining

To observe an immunohistochemical change in large intestine tissues, each slide was reacted with antibody MX2 to calculate a site at which MX2 was expressed in the tissues.

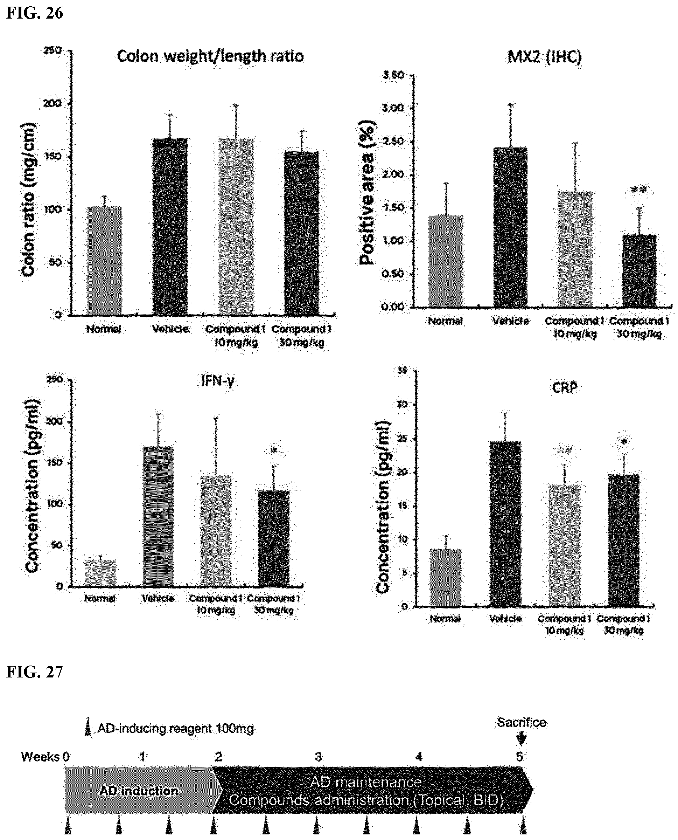

The results of the evaluation methods (1) to (3) are shown in FIG. 26. In FIG. 26, "Compound 1" means Compound 1 (freebase) obtained according to Preparation Example 1.

Referring to FIG. 26, it can be confirmed that Compound 1, which is a test material, exhibits efficacy in an IBD rat model. It can be confirmed that the group dosed with Compound 1 produces a significantly lower level of proinflammatory cytokines compared to the control group (vehicle).

### Experimental Example 10 - HDM-induced model

### 1. Induction of atopy model and treatment with test material (FIG. 27)

### (1) First-time induction method

After the back of the ears and neck of the NC/Nga mice was shaved with a razor, a proper amount of depilatory was applied and depilation was performed. After the depilatory was wiped out, about 100 mg of an atopic dermatitis-inducing reagent (Biostir, Japan) was uniformly applied to the back of the ears and neck using a micro pipette tip.

### (2) Induction method after two occasions

After hair removal was performed using a razor as needed, 150 µL of 4% SDS aqueous solution was uniformly applied to the back of the ears and neck using a micro pipette. After drying with cold air using a dryer, the solution was naturally dried for about two to three hours. About 100 mg of the AD-inducing reagent was uniformly applied to the back of the ears and neck using a micro pipette tip. All treatments were performed 11 times twice a week for a total of six weeks.

### (3) Preparation of administered test material

As a test material, an appropriate amount of phosphate in Compound 1 obtained according to Example 3 was weighed and then prepared by dissolving in a local vehicle (acetone:DMSO=7: 1 v/v). At the time of preparation and administration, the test material was maintained in a light-shielding state, and was prepared and used every three days under the conditions of light-shielding refrigeration.

### (4) Administration

The administration of the test material was started from the time when the atopic score reached 2.1 to 2.3 points, and was directly applied to the center of the skin twice/day until the day of autopsy from the start date of administration of the test material (Day 0) for three weeks.

### 2. Autopsy

The AD-inducing material was applied and sensitized 24 hours before the autopsy, and the drug was administered and applied one hour before the autopsy. 24 hours after induction of atopic dermatitis, the animals were anesthetized. When anesthesia was confirmed, the animals were opened and blood was collected from the caudal vena cava using a syringe. After blood collection, the animal was euthanized, and the induced site (skin and ears) was removed. The extracted skin was divided into two, half of which was fixed in a 10% neutral buffered formalin solution, and the other half was stored in a cryogenic freezer (about -70°C) until analysis.

### 3. Evaluation method

### (1) Clinical atopic dermatitis score

To evaluate atopic dermatitis, evaluation of a skin clinical index (Matsuda et al., 1997) was performed from week 0 of the onset of atopic dermatitis. The evaluation of the skin clinical index (Matsuda et al., 1997) was performed as follows: erythema/hemorrhage, edema, excoriation/erosion, and scaling/dryness were rated as none (0), mild (1), moderate (2), and severe (3), but the rating was based on the Biostir visual evaluation. After that, the sum of the scores for each item was evaluated as a final grade.

### (2) ELISA analysis

An IgE concentration in blood was analyzed with serum obtained by centrifuging the blood collected on the day of autopsy, and IL-1β, IL-4, IL-13, TNF-α, IL-6, and IL-31 were analyzed using the extracted skin. Analysis was performed using a commercially available kit.

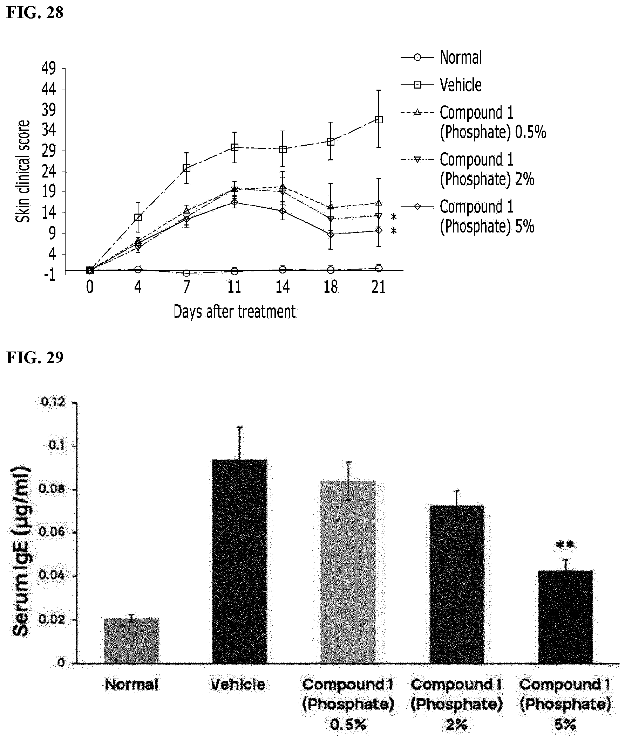

The results thereof were shown in FIGS. 28 to 30. In FIGS. 28 to 30, * p<0.05, ** p<0.01, and *** p<0.001 (n=8). In FIGS. 28 to 30, "Compound 1 (phosphate)" means the "phosphate compound" obtained according to Example 3.

FIG. 28 is a graph showing the results of clinical atopic dermatitis score in Experimental Example 10, and it can be confirmed that skin severity scores of all groups dosed with the phosphate compound are significantly reduced.

FIG. 29 is a graph showing an IgE concentration in plasma of Experimental Example 10. Referring to FIG. 29, it can be confirmed that the serum IgE levels of the groups dosed with Compound 1 are decreased compared to the control group.

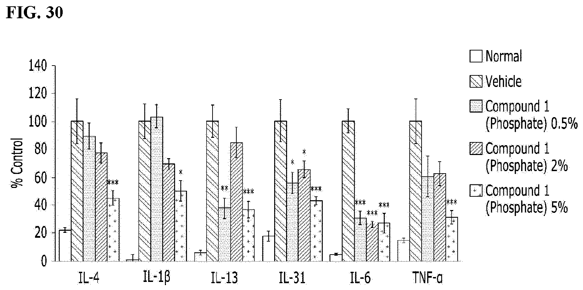

FIG. 30 shows the results of analysis of atopic dermatitis-related cytokines in Experimental Example 10. Referring to FIG. 30, it can be confirmed that the groups dosed with Compound 1 show a decrease in atopic dermatitis-related cytokines compared to the control group.

## Claims

1. A pharmaceutically acceptable salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, wherein the pharmaceutically acceptable salt is hydrochloride salt, hydrobromide salt, phosphate salt, camsylate salt, or oxalate salt.

2. The pharmaceutically acceptable salt of claim 1, wherein the pharmaceutically acceptable salt is hydrochloride salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

3. The pharmaceutically acceptable salt of claim 1, wherein the pharmaceutically acceptable salt is hydrobromide salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

4. The pharmaceutically acceptable salt of claim 1, wherein the pharmaceutically acceptable salt is phosphate salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

5. The pharmaceutically acceptable salt of claim 1, wherein the pharmaceutically acceptable salt is camsylate salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

6. The pharmaceutically acceptable salt of claim 1, wherein the pharmaceutically acceptable salt is oxalate salt of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

7. The pharmaceutically acceptable salt of any one of claims 1 to 6, wherein N-(4-(1-(2-cyanoacetyl)-3-methyl-1,2,3,6-tetrahydropyridin-4-yl)-1H-pyrrolo[2,3-b]pyridin-6-yl)cyclopropanecarboxamide and pharmaceutically acceptable salt are comprised at an equivalence ratio of 1:1 to 1:1.3.

## Patentansprüche

1. Pharmazeutisch verträgliches Salz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid, wobei das pharmazeutisch verträgliche Salz Hydrochloridsalz, Hydrobromidsalz, Phosphatsalz, Camsylatsalz oder Oxalatsalz ist.

2. Pharmazeutisch verträgliches Salz nach Anspruch 1, wobei das pharmazeutisch verträgliche Salz Hydrochloridsalz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid ist.

3. Pharmazeutisch verträgliches Salz nach Anspruch 1, wobei das pharmazeutisch verträgliche Salz Hydrobromidsalz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid ist.

4. Pharmazeutisch verträgliches Salz nach Anspruch 1, wobei das pharmazeutisch verträgliche Salz Phosphatsalz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid ist.

5. Pharmazeutisch verträgliches Salz nach Anspruch 1, wobei das pharmazeutisch verträgliche Salz Camsylatsalz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid ist.

6. Pharmazeutisch verträgliches Salz nach Anspruch 1, wobei das pharmazeutisch verträgliche Salz Oxalatsalz von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid ist.

7. Pharmazeutisch verträgliches Salz nach einem der Ansprüche 1 bis 6, wobei N-(4-(1-(2-Cyanoacetyl)-3-methyl-1,2,3,6-tetrahydropyridin-4-yl)-1H-pyrrolo[2,3-b]pyridin-6-yl)cyclopropancarboxamid und pharmazeutisch verträgliches Salz in einem Äquivalenzverhältnis von 1:1 bis 1:1,3 umfasst sind.

## Revendications

1. Sel acceptable pharmaceutiquement de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide, dans lequel le sel acceptable pharmaceutiquement est un sel chlorhydrate, un sel bromhydrate, un sel phosphate, un sel camsylate ou un sel oxalate.

2. Sel acceptable pharmaceutiquement de la revendication 1, dans lequel le sel acceptable pharmaceutiquement est un sel chlorhydrate de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide.

3. Sel acceptable pharmaceutiquement de la revendication 1, dans lequel le sel acceptable pharmaceutiquement est un sel bromhydrate de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide.

4. Sel acceptable pharmaceutiquement de la revendication 1, dans lequel le sel acceptable pharmaceutiquement est un sel phosphate de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide.

5. Sel acceptable pharmaceutiquement de la revendication 1, dans lequel le sel acceptable pharmaceutiquement est un sel camsylate de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide.

6. Sel acceptable pharmaceutiquement de la revendication 1, dans lequel le sel acceptable pharmaceutiquement est un sel oxalate de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide.

7. Sel acceptable pharmaceutiquement de l'une quelconque des revendications 1 à 6, dans lequel le N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide et le sel acceptable pharmaceutiquement sont présents selon un rapport d'équivalence de 1:1 à 1:1,3.
